# EUROPÄISCHE PATENTSCHRIFT

(11) **EP 1 248 596 B1**
(45) Veröffentlichungstag und Bekanntmachung des Hinweises auf die Patenterteilung: **07.03.2007**
(21) Anmeldenummer: 01909488.7
(22) Anmeldetag: 11.01.2001
(51) Int. Cl.: A61K 9/10

(54) **KIT ZUR IMPLANTATION ENTHALTEND EINE TRÄGERPHASE UND EIN LÖSUNGSMITTEL**
IMPLANTATION KIT COMPRISING A SUPPORT PHASE AND A SOLVENT
KIT A IMPLANTATION COMPRENANT UNE PHASE DE SUPPORT ET UN SOLVENT

(30) Priorität: 11.01.2000 DE 10001683; 11.01.2000 DE 10001682
(43) Veröffentlichungstag der Anmeldung: 16.10.2002
(73) Patentinhaber: Bodmeier, Roland, 14163 Berlin (DE)
(72) Erfinder: Bodmeier, Roland, 14163 Berlin (DE)
(74) Vertreter: Bohmann, Armin K.
(86) Internationale Anmeldenummer: PCT/DE2001/000179
(87) Internationale Veröffentlichungsnummer: WO 2001/051024

(56) Entgegenhaltungen:
- EP-A- 0 649 622
- WO-A-98/55100
- US-A- 5 081 156
- US-A- 5 595 760

## Beschreibung

Die vorliegende Erfindung betrifft Kits zur Bildung von Implantaten und/oder Partikel.

Für die Behandlung bestimmter Krankheiten kann der Arzneistoff als Depotform parenteral appliziert werden. Dazu bieten sich neben klassischen Arzneiformen, wie z.B. öligen Suspensionen, moderne Arzneiformen auf der Basis biokompatibler/bioabbaubarer Polymere an. Da die polymeren Träger fest sind, werden diese in entsprechende Implantate (Ein-Körper-System) oder Mikropartikel (multipartikuläre Systeme) verarbeitet und dann in den Körper durch Implantation oder Injektion eingebracht.

Zur Implantatherstellung wird der Arzneistoff mit dem Träger (z.B. einem Polymer) gemischt und dann in die gewünschte Implantatform (z.B. Zylinder, Pellet, Film, Faser), z.B. durch Extrusion oder Verpressen bei erhöhten Temperaturen gebracht. Solche festen Implantate werden dann meist durch einen chirurgischen Eingriff oder durch Hohlnadeln mit großem Durchmesser in den Körper eingebracht.

Um solche chirurgischen Eingriffe bei Implantaten, die von Patienten meist unerwünscht sind, zu umgehen, können arzneistoffhaltige Mikropartikel verwendet werden. Suspensionen dieser Partikel können aus einer Spritze durch eine Injektionsnadel injiziert werden. Diese Mikropartikel werden außerhalb des Körpers durch verschiedene Verfahren wie z.B. Lösungsmittelverdampfungs- ("Solvent evaporation"), organische Phasenseparations- oder Sprühtrocknungs-Verfahren hergestellt. In der zur Herstellung bioabbaubarer Mikropartikel häufig verwendeten Solvent-Evaporations-Methode wird z.B. ein Arzneistoff in einer Lösung eines bioabbaubaren Polymeren, wie z.B. Polymilchsäure, in einem mit Wasser nicht mischbaren Lösungsmittel (z.B. Dichlormethan) gelöst oder dispergiert. Diese arzneistoffhaltige Polymerphase wird dann in eine äußere wäßrige Phase zur Bildung wirkstoffhaltiger Polymertröpfchen emulgiert. Die Mikropartikel werden nach Verdampfen des Lösungsmittels durch Erhärten des Polymeren erhalten und dann von der wäßrigen Phase z.B. durch Filtration abgetrennt und getrocknet.

Handelspräparate bioabbaubarer Mikropartikel (z.B. Decapeptyl, Enantone) bestehen aus einem Trockenpulver der Mikropartikel und einem wäßrigen Suspensionsvehikel. Aufgrund der hydrolytischen Instabilität der bioabbaubaren Polymeren werden die Mikropartikel und das wäßrige Suspensionsvehikel getrennt, z.B. in Zweikammerspritzen oder in zwei Ampullen, aufbewahrt. Die Mikropartikel werden dann unmittelbar vor der Anwendung in dem wäßrigen Suspensionsvehikel suspendiert und dann injiziert.

Die Herstellung dieser bioabbaubaren Partikelpräparate ist sehr aufwendig und muß unter sterilen oder aseptischen Bedingungen erfolgen. Weiterhin sind die meisten Mikroverkapselungsverfahren nur schwer oder gar nicht auf den Produktionsmaßstab zu übertragen und abhängig von vielen Prozeß- und Formulierungsvariablen. Ferner kann die Suspendierung der Mikropartikel mit nachfolgender Injektion mit Schwierigkeiten verbunden sein (z.B. Agglomeration, Mikropartikelrückstände in der Spritze, Verstopfen der Kanüle, etc.).

Neben den wasserunlöslichen Polymilchsäurederivaten und anderen wasserunlöslichen Polymeren können auch hydrophile Polymere als Trägermaterialien für Mikropartikel oder Implantate eingesetzt werden. Mikropartikel aus hydrophilen Polymeren (z.B. Polysaccharide wie Alginate oder Chitosan, Cellulosederivate, Protein-(Kollagen)-derivate) können z.B. durch Sprühtrockung oder w/o-Emulsionsverfahren hergestellt werden, wobei die wirkstoffhaltige wäßrige Polymerlösung entweder sprühgetrocknet oder in eine äußere, ölige Phase emulgiert wird und die Partikel nach Entfernung des Wassers, Waschens, Filtration und Trocknung erhalten werden. Ähnlich wie die Verfahren zur Herstellung von Polymilchsäure-Mikropartikel sind auch die Mikroverkapselungsverfahren mit hydrophilen Polymeren sehr aufwendig.

Um die Probleme bei der Herstellung und Anwendung von Implantaten und Mikropartikeln zu vermeiden, wurden Zubereitungen, bestehend aus einer arzneistoffhaltigen Polymerlösung, entwickelt (US-PS 4,938,763). Dabei wird z.B. eine wirkstoffhaltige Polylactid-co-glycolidlösung in den Körper, z.B. intramuskulär, subkutan oder periodontal injiziert, ein Implantat bildet sich in-situ durch Ausfällen des Polymeren im Gewebe und der Wirkstoff wird dann retardiert freigesetzt. Das Implantat wird also nicht außerhalb sondern erst innerhalb des Körpers gebildet. Dabei ist der Wirkstoff entweder in der Polymerlösung gelöst oder dispergiert oder er wird, im Falle von Stabilitätsproblemen, getrennt von der Polymerlösung aufbewahrt. Die Herstellung dieser Zubereitung ist wesentlich einfacher als die Herstellung von konventionellen Implantaten oder Mikropartikeln.

Ein Handelspräparat basierend auf dieser in-situ Implantat-Technologie (Atrigel-Technologie) ist in den USA bereits im Handel und befindet sich in Europa im Zulassungsverfahren. Dabei handelt es sich um das Präparat mit dem Handelsnamen Atridox, ein Produkt zur periodontalen Anwendung von Doxycyclin. Aus Stabilitätsgründen sind der Arzneistoff Doxycyclin und die Polymerlösung (bestehend aus dem Polymeren, Poly(dl-lactid) gelöst in dem Lösungsmittel, N-Methyl-2-pyrrolidon) getrennt voneinander in zwei Spritzen aufbewahrt. Kurz vor der Anwendung wird der Arzneistoff und die Polymerlösung über ein Verbindungsstück, das die beiden Spritzen verbindet, durch Hin- und Herschieben der Spritzenkolben gemischt und dann appliziert. Dabei ist ein 200-maliges Hin-und Herschieben der Kolben zur guten Verteilung des Arzneistoffes in der Polymerlösung vor Applikation notwendig. Das Handelspräparat muß aus Stabilitätsgründen zusätzlich bei niedrigen Temperaturen gelagert werden.

Die veröffentlichte europäische Patentanmeldung EP 0 649 662 offenbart ein Kit, das eine Vorrichtung zum Ausbilden eines Implantates, einen Abstandshalter, ein Gefäß mit einer Polymerlösung in einem organischen Lösungsmittel und ein Gefäß mit einem wässrigen Medium enthält, wobei das Polymer aus der Polymerlösung nach Kontakt mit dem wässrigen Medium ausfällt.

Ferner wurden einige Systeme entwickelt, bei denen eine Verfestigung/Viskositätserhöhung wirkstoffhaltiger Polymerlösungen nach Applikation/Injektion in den Körper nicht durch Lösungsmitteldiffusion, sondern in erster Linie durch Temperatur- oder pH-Änderung oder durch bestimmte Substanzen (z.B. Ionen) hervorgerufen wird. Diese Systeme haben meist die gleichen Nachteile wie die in den beiden vorhergehenden Paragraphen beschriebenen Systeme.

Die Polymerlösung muß durch eine Nadel injizierbar sein, darf also nicht zu viskos sein. Der Polymergehalt ist damit in erster Linie durch die Viskosität und nicht durch die Löslichkeit des Polymeren limitiert. Auch während der Injektion der Polymerlösung kann durch Ausfällung des Polymeren die Injektion der verbleibenden Polymerlösung negativ beeinflußt werden. Nachteile dieser Methode sind ferner die Verwendung hoher Lösungsmittelanteile mit entsprechender Toxizität oder Verträglichkeitsproblemen und, nach Injektion in das Weichgewebe, die etwas unkontrollierte Verhärtung des Polymeren mit nicht genau definierter Oberfläche des Implantats. Dies kann zu irreproduzierbaren Freisetzungsprofilen führen. Ferner kann der Wirkstoff vor der Verhärtung der Polymerlösung rasch freigesetzt werden. Dieser sogenannte "Burst-Effekt" ist meist unerwünscht.

In WO 98/55100 werden Zubereitungen beschrieben, die einfach herzustellen sind, und die Probleme, die bei der Entwicklung und Anwendung von Mikropartikeln und Implantaten, auch der beschriebenen in-situ Implantate, vermeiden. Dabei handelt es sich um Zubereitungen, die bei Einbringen in einen Körper Partikel und/oder Implantate bilden, und die durch Mischen einer inneren polymerhaltigen Trägerphase mit einer nicht mischbaren oder teilweise nicht mischbaren zweiten äußeren Phase erhältlich sind, wobei sich bei nachfolgender Veränderung der Umgebungsbedingungen die Viskosität der Trägerphase verändert.

Zum Beispiel kann eine wirkstoffhaltige Dispersion, bestehend aus einer inneren Trägerphase und einer zweiten, äußeren Phase (z.B. ein Öl) hergestellt, und in den Körper eingebracht werden. Die innere Trägerphase ist z.B. eine Lösung bestehend aus einem Polymeren (Trägermaterial) und einem Lösungsmittel. Nach Einbringen in den Körper kommt es dann zu einer Verfestigung der inneren Phase, z.B. durch Lösungsmitteldiffusion in die Umgebung oder Eindiffusion von Körperflüssigkeiten, oder durch Änderung der Temperatur, des pH-Wertes oder der Ionenstärke. Die Dispersion kann z.B. im Falle von bioabbaubaren Polymeren i.m. oder s.c. injiziert werden. Im Kontakt mit Körperflüssigkeiten kann sich die innere Phase verfestigen und z.B. Partikel bilden.

In WO 98/55100 wird beschrieben, daß die Trägerphase auch erst kurz vor der Anwendung mit der zweiten, äußeren Phase gemischt werden kann. Dies kann notwendig sein, wenn die Dispersion physikalisch instabil ist, d.h. die innere Trägerphase trennt sich von der zweiten, äußeren Phase.

Die Erfindung wird durch den Gegenstand der unabhängigen Ansprüche beschrieben. Besonders bevorzugte Ausführungsformen ergeben sich aus den Unteransprüchen.

Völlig überraschend wurde im Rahmen dieser Erfindung gefunden, daß auch die Trägerphase erst kurz vor der Anwendung hergestellt werden kann. Das Polymer (Trägermaterial) wird also erst kurz vor der Anwendung mit dem Lösungsmittel gemischt und muß daher nicht in Form einer Polymerlösung, also der fertigen Trägerphase, gelagert werden. Die erfindungsgemäße Zubereitung besteht also nicht aus einer Trägerphase (Polymerlösung, wie das oben beschriebene in-situ Implantat System), sondern z.B. aus einem Polymeren in einer Spritze und einem davon getrennten Lösungsmittel in einer zweiten Spritze, wobei das Polymer in dem Lösungsmittel löslich ist. Das Polymer wird dann erst kurz vor der Anwendung über ein Verbindungsstück, das die beiden Spritzen verbindet, mit dem Lösungsmittel durch Hin- und Herschieben der Spritzenkolben gemischt und in Lösung gebracht.
Diese Mischung wird dann gegebenenfalls in eine weitere flüssige Phase dispergiert (z.B. über ein Verbindungsstück mit einer dritten, die äußere Phase enthaltene Spritze) und dann appliziert. Das Trägermaterial und das Lösungsmittel können auch in einer Zweikammerspritze gemischt werden und anschließend gegebenenfalls über ein Verbindungsstück mit der weiteren flüssigen Phase (die sich in einer zweiten Spritze befindet) dispergiert werden. Viele Mischmöglichkeiten zur Herstellung der Dispersion sind möglich.

Eine getrennte Aufbewahrung des Polymeren und des Lösungsmittels im Endprodukt und damit die Herstellung der Trägerphase erst kurz vor der Anwendung hat viele Vorteile im Vergleich zur Lagerung einer Polymerlösung. Ein wichtiger Punkt dabei ist die Stabilität der Darreichungsform. Dazu zählt u.a. die chemische Stabilität der Wirk- und Hilfsstoffe (z.B. des Polymeren) während der Lagerung. Der Wirkstoff und/oder das Polymer können während der Lagerung in gelöster Form abgebaut werden. Vor allem höhermolekulare Wirkstoffe (z.B. Peptide oder Proteine) oder auch Polymere und andere Hilfsstoffe enthalten häufig nicht vernachlässigbare Mengen an Wasser, die die Stabilität, vor allem auch die Stabilität der biokompatiblen, hydrolytisch abbaubaren Polymere, negativ beeinflußen. Die Molmasse des Polymeren kann sich während der Lagerung in flüssigem Zustand verändern, dies beeinflußt natürlich auch die Wirkstofffreisetzung und kann zu nicht akzeptablen Veränderungen in der Wirkstofffreisetzung und damit auch der pharmakologischen Wirkung führen.

Ein weiterer Vorteil dieser Erfindung ist, daß eine Lagerung bei niedrigen Temperaturen meist nicht notwendig ist.

Ferner kann in einem Arbeitsgang der Wirkstoff und das Polymer in das Lösungsmittel eingearbeitet werden, die aufwendige Herstellung der Polymerlösung entfällt damit.

Neben der chemischen Stabilität spielt bei flüssigen dispersen Systemen, z.B. bei Arzneistoffsuspensionen in den Polymerlösungen, auch die physikalische Stabilität eine wichtige Rolle. Während der Lagerung kann es zur Sedimentation und zur Bildung eines nicht mehr aufschüttelbaren Kuchens kommen.

Es ist daher wünschenswert, die Stabilitätsprobleme dieser flüssigen, wirkstoffhaltigen Trägermateriallösungen zu beseitigen.

Diese Aufgabe wird dadurch gelöst, daß eine Zubereitung für die Bildung von Implantaten und/oder Partikeln zur Verfügung gestellt wird, wobei sie mindestens ein Trägermaterial wobei das Trägermaterial ein Polymer, Lipid oder Wachs ist, und ein vom Trägermaterial getrenntes Lösungsmittel enthält wobei das Trägermaterial in dem Lösungsmittel löslich ist, und das Trägermaterial erst kurz vor der Anwendung mit dem Lösungsmittel gemischt und darin gelöst wird, und dieses Gemisch (gegebenenfalls nach zusätzlichem Dispergieren in eine weitere flüssige, nicht mischbare oder teilweise nicht mischbare äußere Phase) nach Anwendung in oder auf dem Körper ein Implantat und/oder Partikel bildet.

Diese Aufgabe wird dadurch gelöst, daß ein Kit zur Herstellung einer Zubereitung, welche eine Trägerphase enthält, die im Körper, auf dem Körper oder unter physiologischen Umgebungsbedingungen ein Implantat oder Partikel bildet, wobei die Trägerphase zumindest ein Trägermaterial und ein Lösemittel enthält, dadurch gekennzeichnet, dass das Trägermaterial und das Lösungsmittel im Kit getrennt voneinander vorliegen wobei das Trägermaterial in dem Lösungsmittel löslich ist und das Trägermaterial ein Polymer, Lipid oder Wachs ist.

Ein Kit enthält dabei eine Kombination von mindestens 2 Komponenten (z.B. Trägermaterial und Lösungsmittel), die zur Herstellung einer applizierbaren Zubereitung verwendet werden und dazu mit geeigneten Hilfsmitteln zusammengegeben werden. Die einzelnen Komponenten und weiterer Hilfsmitttel (z.B. Spritzen) sind normalerweise in einer Sekundärverpackung verpackt.

Der Begriff Implantat/Partikel ist weitgreifend, z.B. in Bezug auf Form, Größe, Konsistenz, Aufbau und Applikationsort. Sie können eine viskose flüssige und/oder eine halbfeste und/oder eine feste Konsistenz besitzen. Der Begriff Implantat/Partikel wird in dieser Erfindung nicht nur für im Körper, sondern auch für auf dem Körper angewendete Zubereitungen verwendet. Dabei können z.B. Filme durch Aufsprühen gebildet werden.

Das Implantat und/oder Partikel bilden sich aus der Zubereitung nach Körperkontakt, z.B. durch eine Konzentrierung oder Ausfällung des Trägermaterials, durch eine Wegdiffusion des Lösungsmittels, durch Kontakt mit Körperflüssigkeiten, durch eine Temperatur- oder pH-Änderung, durch am Applikationsort vorhandene Substanzen, durch eine Veränderung der Ionenstärke oder - art, oder durch eine Kombination der genannten Faktoren.

Das Implantat und/oder Partikel können auch außerhalb und nicht in Kontakt mit dem Körper gebildet werden. Dabei wird z.B. die erfindungsgemäße Zubereitung nach Mischen des Tägermaterials mit dem Lösungsmittel in eine Form gebracht und in Kontakt mit wässrigen Flüssigkeiten in dieser vorgefertigten Form verfestigt. Dieses extern gebildete Implantat und/oder Partikel können dann in oder auf den Körper gebracht werden.

Erfindungsgemäß bevorzugt ist, daß das Trägermaterial und das Lösungsmittel getrennt in Spritzen oder in einer Zweikammerspritze aufbewahrt sind, und daß das Trägermaterial mit dem Lösungsmittel über ein Verbindungsstück (das die beiden Spritzen verbindet) oder innerhalb der Zweikammerspritze erst kurz vor der Anwendung gemischt und darin gelöst wird.

Erfindungsgemäß kann ferner sein, daß daß das Trägermaterial in fester Form (z.B als Pulver) in eine Spritze oder eine Kammer der Zweikammerspritze gebracht wird, oder als Lösung oder Dispersion in eine Spritze oder in eine Kammer der Zweikammerspritze gebracht wird und anschließend darin, z.B durch Lyophilisation, getrocknet wird.

Das Trägermaterial und das Lösungsmittel können natürlich auch in sonst üblichen Behältnissen (z.B. Ampullen oder Glasvials) getrennt aufbewahrt werden.

Das Trägermaterial bildet das Gerüst des Implantates/Partikel und ist bei wirkstoffhaltigen Zubereitungen für die Retardierung der Wirkstofffreisetzung wichtig.

Erfindungsgemäß bevorzugt ist, dass das Trägermaterial ein wasserlösliches, ein wasserunlösliches Polymer, ein in wässrigen Flüssigkeiten lösliches Polymer, ein biokompatibles und/oder bioabbaubares Polymer, ein Protein, oder ein Lipid, ist.

Erfindungsgemäß bevorzugt ist, daß das Trägermaterial ein Polylaktid oder ein Polylaktid-derivat ist, oder ein Monoglycerid (z.B. Glycerolmonooleat, -linoleat) enthält, daß es nach Anwendung in oder auf dem Körper flüssigkristalline Phasen, z.B. kubische Phasen, bildet.

Die Trägermaterialen sind synthetischen, halbsynthetischen und natürlichen Ursprungs. Dazu zählen ferner Cellulosederivate (z.B. Celluloseacetat, Ethylcellulose, Celluloseacetatphthalat, Celluloseether wie z.B Hydroxypropylmethylcellulose), Acrylatderivate (z.B. Eudragite, Poly(methylmethacrylat), Cyanoacrylate) und biokompatible und bioabbaubare Polymere wie Polyanhydride, Polyester, Polyorthoester, Polyurethane, Polycarbonate; Polyphosphazene, Polyacetale, Polyoxyethylen-oxypropylene. Wichtig dabei sind Polyester wie Polylaktid, Polyglykolid, Polycaprolacton, Polyhydroxybutyrat-oder valerat. Ferner können auch Polysaccharide wie Na Alginate, Chitosan oder Chitin oder Proteine verwendet werden. Ein Vielzahl von Trägermaterialien sind in der Literatur beschrieben, diese kommen potentiell alle für die erfindungsgemäßen Zubereitungen in Frage.

Erfindungsgemäß kann ferner sein, daß das Lösungsmittel Wasser oder ein organisches Lösungsmittel, wie Ethanol, Aceton, Butanol, Ethylformat, Essigsäure Pentanol, n- oder i-Propanol, Tetrahydrofuran, Triethylcitrat, Triacetin, Propylenglykol, Glycerol, Polyethylenglykol, Aceton, Ethylacetat, Methylacetat, Ethyllactat, Benzylalkohol, Dimethylformamid, Dimethylsulfoxid, Dimethylacetamid, Propylencarbonat, Glykofurol, Ölsäure, 2-Pyrrolidon, N-Methyl-2-Pyrrolidon ist, oder ein Öl oder ein Weichmacher oder eine Mischung aus zwei oder mehreren der genannten Lösungsmittel ist.

Es bieten sich vor allen auch Lösungsmittel aus der Draft guideline of the international conference on harmonization on impurities residual solvents an. Das Lösungsmittel löst das Trägermaterial. Es können selbstverständlich auch Kombinationen aus Lösungsmitteln und Nichtlösungsmitteln für das Trägermaterial verwendet werden. Es können auch Lösungsmittelgemische verwendet werden, ausgewählt z.B. nach Lösungsmittelqualität für das Trägermaterial oder Mischbarkeit mit wässrigen Phasen. Durch die Lösungsmittelauswahl kann z.B. die Auflösungsgeschwindigkeit des Trägermaterials und die Mischbarkeit mit Körperflüssigkeiten beeinflusst werden. Das Lösungsmittel sollnicht oder nur teilweise mit der weiteren flüssigen Phase mischbar sein. Die Viskosität der Trägerphase kann durch das Trägermaterial (z.B. Molmasse, Konzentration etc.) und auch das Lösungsmittel beeinflusst werden. Der Verfestigungsgrad nach Applikation einer erfindungsgemäßen Zubereitung kann durch die Auswahl des Trägermaterials und des Lösungsmittels beeinflusst werden.

Die Auflösungsgeschwindigkeit des Trägermaterials in dem Lösungsmittel hängt von vielen Faktoren ab, z.B. von der Lösungsmittelqualität für das Trägermaterial, von der Teilchengröße, der Molmasse, der Porosität und der Konzentration des Trägermaterials, von der Temperatur und der Mischintensität. Das Trägermaterial kann durch verschiedene Verfahren in Pulverform erhältlich sein. Poröse Teilchen können z.B. durch Sprühtrocknung von Trägermateriallösungen (z.B. Polymerlösungen) erhalten werden. Kleinere. Teilchen können auch durch Vermählen erhalten werden. Lyophilisierung von Polymerlösungen ergibt einen porösen Schwamm (z.B. in der Spritze oder Zweikammerspritze) oder ein poröses Pulver. Nach Zugabe des Lösungsmittels löst sich dieser Schwamm aufgrund seiner geringen Dichte sehr schnell auf. Die erfindungsgemäße Zubereitung kann daher schnell appliziert werden.

Erfindungsgemäß kann ferner sein, daß die weitere flüssige Phase mit wäßrigen Flüssigkeiten vollständig, teilweise oder nicht mischbar ist, und daß die weitere flüssige Phase getrennt vom dem Trägermaterial und dem Lösungsmittel, in Kontakt mit dem Trägermaterial und getrennt vom Lösungsmittel, in Kontakt mit dem Lösungsmittel und getrennt vom Trägermaterial oder in einer Kombination der oben genannten Möglichkeiten aufbewahrt wird.

Erfindungsgemäß kann ferner sein, daß die weitere flüssige Phase natürliche, halbsynthetische oder synthetische Fette, Öle oder Wachse, wie Baumwollensaatöl, Sojaöl, Saffloröl, hydriertes Erdnußöl, Olivenöl, Rizinusöl, Triglyceridgemische (wie Miglyol), Monoglyceride (wie Glycerolmonooleat) Silikonöl, Isopropylmyristat, Ethyloleat, Paraffine, Wasser, Glycerol, Propylenglycol oder Polyethylenglycol oder Mischungen aus zwei oder mehreren der oben genannten Stoffe umfaßt.

Erfindungsgemäß kann ferner sein, daß die Zubereitung einen Wirkstoff umfasst, daß der Wirkstoff ein Arzneistoff, ein Peptid- oder Proteinarzneistoff, ein.Oligonucleotid oder ein Gentherapeutikum ist, daß der Arzneistoff aus der Gruppe der Antibiotika, Antiinflammatoria, Antiinfektiva, Hormone, immunologisch wirksame Stoffe, Impfstoffe, Immunmodulatoren, Immunsuppresiva, zytostatika, Diuretika, Magen-Darm-Mittel, Herz-Kreislauf-Mittel, Respirationstrakt-Mittel, Wachstumsfaktoren Analgetika, Lokalanaesthetika und/oder Neuropharmaka stammt, oder daß der Arzneistoff Tetracyclin oder Doxycyclin ist.

Zu den Wirkstoffen zählen nieder- und höhermolekulare Arzneistoffe zur human- und veterinärmedizinischen Anwendung und Substanzen, die in der Landwirtschaft, im Haushalt, in der Nahrungsmittel-, kosmetischen und chemischen Industrie und anderen Industriezweigen genutzt werden.

Die erfindungsgemäße Zubereitung bewirkt, daß der Wirkstoff am Applikationsort verzögert freigegeben wird. Die Wirkstofffreisetzung kann unter anderem durch den Disperisitätsgrad, die Wirkstoffbeladung, das Trägermaterial und die Trägermaterialkonzentration beeinflusst werden. Ferner können auch Freigaberegulatoren, wie z.B. hydrophile oder lipophile Stoffe anorganischer, organischer oder polymerer Natur mit eingearbeitet werden. Ein besonderer Vorteil der Dispersionen ist, daß im Vergleich zu wirkstoffhaltigen Polymerphasen (also keine Dispersionen), die in-situ ein Implantat bilden, eine anfängliche, rasche Freigabe des Wirkstoffes am Applikationsort vermieden wird. Die wirkstoffhaltige Trägerphase stellt die innere Phase der Zubereitung dar und ist daher nach der Applikation überwiegend nicht sofort in Kontakt mit dem umgebenden Körper.

Erfindungsgemäß kann ferner sein, daß der Wirkstoff verkapselt ist. Der Wirkstoff kann in Partikel verkapselt vorliegen. Der Begriff 'Partikel' in diesem Zusammenhang (zu unterscheiden von Partikeln, die aus der Trägerphase gebildet werden) ist weitgreifend und umfaßt z.B. Mikropartikel, Mikrokapseln, kolloidale Teilchen (z.B Nanopartikel, Nanokapseln, Liposomen), an partikuläre Ionenaustauscherharze gebundene Wirkstoffe, Cyclodextrine.

Das Verkapselungsmaterial kann polymerer als auch nichtpolymerer Herkunft (z.B. Lipide) sein. Durch die Kombination von Partikel und Implantat kann z.B die Retardierung der Freisetzung verlängert werden oder der Burst-Effekt (anfänglich rasche Freisetzung) verringert/eliminiert werden. Das Lösungmittel für das Trägermaterial des Implantats sollte das Verkapselungsmaterial nicht oder langsamer als das Trägermaterial lösen. Verkapselungs- und Trägermaterial können aus einer chemisch gleichen Substanzgruppe kommen, sie unterscheiden sich z.B. im Falle von Polymeren in der Molmasse oder in dem Monomerenverhältnis.

Durch Einarbeitung des verkapselten Wirkstoffes (z.B. Mikropartikel) in eine erfindungsgemäße Zubereitung kann z.B. auch eine häufig unerwünschte zu rasche anfängliche Wirkstofffreisetzung vermieden werden.

Erfindungsgemäß kann ferner sein, daß der Wirkstoff mit dem Trägermaterial gemischt vorliegt oder in dem Lösungsmittel/Lösungsmittelgemisch gelöst und/oder dispergiert ist, oder in beiden vorliegt. Ein pulverförmiger Wirkstoff kann z.B. zusammen mit dem pulverförmigen Trägermaterial in das Behältnis (z.B. eine Spritze) abgefüllt werden, sowohl Wirkstoff als auch Trägermaterial liegen dann in fester Form vor. Der Wirkstoff kann auch in eine Lösung des Trägermaterials gelöst oder dispergiert werden, diese Flüssigkeit wird dann in das Behältnis abgefüllt und getrocknet, z.B. durch Lyophilisation. In diesen Fällen liegt der Wirkstoff also zusammen mit dem Trägermaterial getrennt von dem Lösungsmittel vor. Vor allem bei der gleichzeitigen Lyophilisation einer flüssigen Wirkstoff-Trägermaterialphase ist der Wirkstoff gleichmäßig in das Trägermaterial eingearbeitet und resultiert in einer schnellen, gleichmäßigen Verteilung nach Zugabe des Lösungsmittels. Alternativ kann der Wirkstoff auch nur in dem Lösungsmittel oder sowohl mit dem Lösungsmittel und dem Trägermaterial aufbewahrt werden.

Erfindungsgemäß kann ferner sein, daß die Zubereitung viskositätsbeeinflussende Substanzen, Stabilisatoren, Freigaberegulatoren, Porenbildner, Substanzen, welche die Verweildauer am Applikationsort verändern, bioadhäsive Materialien und/oder Penetrationsverbesserer enthält, wobei der Wirkstoff am Applikationsort verzögert freigegeben und/oder eine anfängliche, rasche Freigabe des Wirkstoffes am Applikationsort vermieden wird. Zur Herstellung der Dispersion mit einer weiteren flüssigen Phase können Stabilisatoren, wie z.B. Emulgatoren notwendig sein. Zu den Emulgatoren zählen unter anderem Polyethylenglykolfettsäureester, -fettsäureether, -sorbitanfettsäureester, Sorbitanfettsäureester, Partialfettsäureester mehrwertiger Alkohole oder Zucker, Lecithine und Poloxamere.

Erfindungsgemäß sind ferner Zubereitung zur parenteralen (z.b. im, sc, intraarteriell), peroralen, subcutanen, rektalen, buccalen, ophthalmischen, pulmonalen, vaginalen, nasalen, lokalen, sublingualen, peridontalen oder transdermalen Anwendung und/oder zur Einbringung in Körperöffnungen und/oder zum Aufbringen auf Körperflächen. Die Zubereitung kann auch zur Behandlung von Weich-und Hartgewebedefekten, z.B. als Gerüst, zur Gewebeerneuerung (tissue regeneration), als Klebstoff, oder zur Füllung von Körperkavitäten, oder Behandlung von Wunden eingesetzt werden. Im Falle der inneren oder externen Wundbehandlung kann die erfindungsgemäße Zubereitung vor der Anwendung auf verschiedene medizinisch verwendete Stoffe/Gewebe aufgebracht werden.

Die Zubereitung kann durch dem Fachmann bekannte Verfahren in Körperöffnungen oder auf Körperflächen aufgebracht werden (z.B. durch Injektion oder Auf-/Einsprühen).

Das erfindungsgemäße Kit besteht also aus mindestens zwei Komponenten in mindestens einer Verpackung zum gemeinsamen Gebrauch.

Der Kernpunkt der Erfindung ist, daß die vom Hersteller (z.B. Arzneimittelhersteller) abgegebene Zubereitung das Polymer in fester und nicht bereits in gelöster Form enthalten wird. Das Polymer wird getrennt vom Lösungsmittel aufbewahrt, z.B. in einer Zweikammerspritze oder in zwei getrennten Spritzen oder Ampullen. Die Polymerlösung (und gegebenenfalls die Dispersion in eine weitere flüssige Phase) wird also nicht vom Hersteller zubereitet, sondern erst vom Anwender (z.B. medizinisches Fachpersonal) kurz vor der Applikation am Patienten.

Bei den bisher vorhandenen Systemen wird das Polymer auch gelöst, jedoch nicht erst kurz vor der Applikation. Das Handelsprodukt Atridox^{®} besteht aus einer Polymerlösung, während bei der erfindungsgemäßen Zubereitung das Handelspräparat aus einem Trägermaterial und einem davon getrennten Lösungsmittel bestehen wird.

Durch die nachfolgenden Beispiele wird die Erfindung erläutert, soll dadurch jedoch nicht eingeschränkt werden.

### Beispiel 1

Poly(d,1-lactid) in Pulverform und das Lösungsmittel 2-Pyrrolidon werden getrennt in zwei Spritzen gefüllt. Nach Verbinden der Spritzen über ein Verbindungsstück und Hin-und Herschieben der Spritzenkolben wird das Polymer zumindest teilweise gelöst. Alternativ kann diese Trägerphase auch innerhalb einer Mehrkammerspritze hergestellt werden. In Kontakt mit Puffer pH 7,4 kommt es zur Verfestigung der Polymerlösung und zur Ausfällung.

Die obige Trägerphase (in einer Spritze) kann auch in eine äußere Erdnußölphase(eventuell stabilitsatorhaltig) (zweite Spritze) über ein Verbindungsstück dispergiert werden. In Kontakt mit Puffer pH 7,4 kommt es zur Verfestigung der Trägerphase.

### Beispiel 2

Alternativ kann Poly(d,1-lactid) (PLA) in fester Form durch Lyophilisation einer organischen PLA-Lösung (z.B. in DMSO) innerhalb einer Spritze erhalten werden und wird dann getrennt vom Lösungsmittel 2-Pyrrolidon aufbewahrt. Nach Verbinden der Spritzen über ein Verbindungsstück und Hin-und Herschieben der Spritzenkolben wird das Polymer zumindest teilweise gelöst. Alternativ kann diese Trägerphase auch innerhalb einer Mehrkammerspritze hergestellt werden. In Kontakt mit Puffer pH 7,4 kommt es zur Verfestigung der Polymerlösung und zur Ausfällung.

Die obige Trägerphase (in einer Spritze) kann auch in eine äußere Erdnußölphase(eventuell stabilitsatorhaltig) (zweite Spritze) über ein Verbindungsstück dispergiert werden. In Kontakt mit Puffer pH 7,4 kommt es zur Verfestigung der Trägerphase.

### Beispiel 3

wie Beispiel 1 oder 2, als Lösungsmittel wird jedoch Ethylacetat und als äußere Phase eine wäßrige Phase verwendet.

### Beispiel 4

Poly(oxyethylen-oxypropylen) (Lutrol F 127, BASF) Pulver (alternativ kann Poly(oxyethylen-oxypropylen) durch Lyophilisation einer wässrigen Poly(oxyethylenoxypropylen)-lösung in der Spritze in die feste Form überführt werden) und Wasser werden getrennt in zwei Spritzen gefüllt. Nach Verbinden der Spritzen über ein Verbindungsstück und Hin- und Herschieben der Spritzenkolben wird das Polymer zumindest teilweise gelöst. Alternativ kann diese Trägerphase auch innerhalb einer Mehrkammerspritze hergestellt werden. Bei Temperaturerhöhung auf 37 °C (z.B. nach Injektion) kommt es zu einer Erhöhung der Viskosität der Polymerphase.

Die obige Trägerphase (in einer Spritze) kann auch in eine äußere Erdnußölphase(eventuell stabilitsatorhaltig) (zweite Spritze) über ein Verbindungsstück dispergiert werden. Bei Temperaturerhöhung auf 37 °C kommt es zu einer Erhöhung der Viskosität der Trägerphase.

### Beispiel 5

Chitosan oder ein Chitosansalz (z.B. Chitosanglutamat) in Pulverform und eine wässrige Phase (leicht angesäuert im Falle von Chitosan) werden getrennt in zwei Spritzen gefüllt. Alternativ kann Chitosan durch Lyophilisation einer wässrigen Chitosanlösung in der Spritze in die feste Form überführt werden. Nach Verbinden der Spritzen über ein Verbindungsstück und Hin- und Herschieben der Spritzenkolben wird das Polymer zumindest teilweise gelöst. In Kontakt mit Puffer pH 7,4 kommt es zur Ausfällung von Chitosan.

Die obige Trägerphase (in einer Spritze) kann auch in eine äußere Erdnußölphase(eventuell stabilitsatorhaltig) (zweite Spritze) über ein Verbindungsstück dispergiert werden. In Kontakt mit Puffer pH 7,4 kommt es zum Ausfällen von Chitosan.

### Beispiel 6

Ein Monoglycerid (Glycerolmonoleat, -linoleat, Myverol 18-92 oder 18-99, oder GMOrphic80) und ein Wasser-Ethanol Gemisch werden getrennt in zwei Spritzen gefüllt. Nach Verbinden der Spritzen über ein Verbindungsstück und Hin-und Herschieben der Spritzenkolben wird das Monoglycerid zumindest teilweise gelöst. In Kontakt mit Puffer pH 7,4 kommt es zur Bildung von viskosen flüssigkristallinen Phasen, z.B. einer kubischen Phase.

### Beispiel 7

Der Wirkstoff (Doxycyclin) wird in Pulverform der Trägermaterialphase der obigen Beispiele beigegeben, oder zusammen mit einer Lösung des Trägermaterials der obigen Beispiele durch Lyophilisation getrocknet.

## Patentansprüche

1. Kit zur Herstellung einer Zubereitung, welche eine Trägerphase enthält, die im Körper, auf dem Körper oder unter physiologischen Umgebungsbedingungen ein Implantat oder Partikel bildet, wobei die Trägerphase zumindest ein Trägermaterial und ein Lösungsmittel enthält, **dadurch gekennzeichnet, dass** das Trägermaterial ein Polymer, Lipid oder Wachs ist und das Trägermaterial und das Lösungsmittel im Kit getrennt voneinander vorliegen, wobei das Trägermaterial in dem Lösungsmittel löslich ist.

2. Kit nach Anspruch 1, **dadurch gekennzeichnet, dass** das Trägermaterial und das Lösungsmittel im Kit in getrennten Spritzen oder in getrennten Kammern einer Zweikammerspritze aufbewahrt sind, und dass das Trägermaterial mit dem Lösungsmittel über ein Verbindungsstück oder innerhalb der Zweikammerspritze vor der Anwendung gemischt und darin gelöst wird.

3. Kit nach einem der Ansprüche 1 bis 2, **dadurch gekennzeichnet, dass** das Trägermaterial ein wasserlösliches Polymer, ein wasserunlösliches Polymer, ein in wässrigen Flüssigkeiten lösliches Polymer, ein biokompatibles und/oder bioabbaubares Polymer, ein Protein, oder eine Kombination der vorgenannten Trägermaterialien ist.

4. Kit nach Anspruch 3, **dadurch gekennzeichnet, dass** das Trägermaterial Polylaktid, ein Derivat oder Copolymer des Polylaktid enthält.

5. Kit nach einem der vorangehenden Ansprüche, **dadurch gekennzeichnet, dass** das Lösungsmittel Wasser oder ein organisches Lösungsmittel wie Ethanol, Aceton, Butanol, Ethylformat, Essigsäure, Pentanol, n-Propanol, Isopropanol, Tetrahydrofuran, Triethylcitrat, Triacetin, Propylenglykol, Glycerol, Polyethylenglykol, Ethylacetat, Methylacetat, Ethyllactat, Benzylalkohol, Dimethylformamid, Dimethylsulfoxid, Dimethylacetamid, Propylencarbonat, Glykofurol, Ölsäure, 2-Pyrrolidon, N-Methyl-2-pyrrolidon, ein Öl, ein Weichmacher oder eine Mischung aus zwei oder mehreren der genannten Lösungsmittel ist.

6. Kit nach einem der vorangehenden Ansprüche, **dadurch gekennzeichnet, dass** die Zubereitung mindestens einen Wirkstoff enthält.

7. Kit nach Anspruch 6, **dadurch gekennzeichnet, dass** der Wirkstoff ein Arzneistoff, ein Peptid- oder Proteinarzneistoff, ein Oligonucleotid oder ein Gentherapeutikum ist.

8. Kit nach einem der Ansprüche 6 bis 7, **dadurch gekennzeichnet, dass** der Wirkstoff ein Arzneistoff aus der Gruppe der Antibiotika - z. B. Tetracyclin oder Doxycyclin-, Antiinflammatoria, Antiinfektiva, Hormone, immunologisch wirksamen Stoffe, Impfstoffe, Immunmodulatoren, Immunsuppressiva, Zytostatika, Diuretika, Magen-Darm-Mittel, Herz-Kreislauf-Mittel, Respirationstrakt-Mittel, Wachstumsfaktoren, Analgetika, Lokalanästhetika und/oder Neuropharmaka ist.

9. Kit nach einem der Ansprüche 6 bis 8, **dadurch gekennzeichnet, dass** der Wirkstoff in der Zubereitung verkapselt vorliegt.

10. Kit nach einem der Ansprüche 6 bis 9, **dadurch gekennzeichnet, dass** der Wirkstoff im Kit mit dem Trägermaterial gemischt oder mit dem Lösungsmittel gelöst und/oder dispergiert oder in beiden vorliegt.

11. Kit nach einem der vorangehenden Ansprüche, **dadurch gekennzeichnet, dass** die Zubereitung viskositätsbeeinflussende Substanzen, Stabilisatoren, Freigaberegulatoren, Porenbildner, Substanzen, welche die Verweildauer am Applikationsort verändern, bioadhäsive Materialien, Penetrationsverbesserer, Substanzen zu Verzögerung der Freigabe und/oder Substanzen zur Vermeidung einer raschen, anfänglichen Freigabe am Applikationsort enthält.

12. Kit nach einem der vorangehenden Ansprüche, **dadurch gekennzeichnet, dass** die Zubereitung zur parenteralen, peroralen, subcutanen, rektalen, bukkalen, ophthalmischen, pulmonalen, vaginalen, nasalen, lokalen, sublingualen, peridontalen oder transdermalen Anwendung, zum Einbringen in Körperöffnungen und/oder zum Aufbringen auf Körperoberflächen bestimmt oder geeignet ist.

13. Kit nach einem der vorangehenden Ansprüche, **dadurch gekennzeichnet, dass** die Zubereitung zur Behandlung von Weich- und Hartgewebedefekten, zur Gewebeerneuerung, als Klebstoff, zur Füllung von Körperkavitäten oder zur Behandlung von Wunden bestimmt oder geeignet ist.

14. Kit nach einem der vorangehenden Ansprüche, **dadurch gekennzeichnet, dass** die Zubereitung außer der Trägerphase eine weitere flüssige Phase enthält, in welcher die Trägerphase dispergiert vorliegt.

15. Kit nach Anspruch 14, **dadurch gekennzeichnet, dass** die weitere flüssige Phase im Kit getrennt vom Trägermaterial und dem Lösungsmittel, in Kontakt mit dem Trägermaterial und getrennt vom Lösungsmittel, in Kontakt mit dem Lösungsmittel und getrennt vom Trägermaterial oder in einer Kombination der genannten Möglichkeiten aufbewahrt wird.

16. Kit nach einem der Ansprüche 14 und 15, **dadurch gekennzeichnet, dass** die weitere flüssige Phase natürliche, halbsynthetische oder synthetische Fette, Öle oder Wachse wie Baumwollsaatöl, Sojaöl, Saffloröl, hydriertes Erdnussöl, Olivenöl, Rizinusöl, Triglyceridgemische (z. B. Miglyol), Monoglyceride (z. B. Glycerolmonooleat), Silikonöl, Isopropylmyristat, Ethyloleat, Paraffine, Wasser, Glycerol, Propylenglykol, Polyethylenglykol oder Mischungen aus zwei oder mehreren der genannten Stoffe enthält.

17. Kit nach einem der Ansprüche 14 bis 16, **dadurch gekennzeichnet, dass** mindestens ein Wirkstoff im Kit mit dem Trägermaterial und/oder mit dem Lösungsmittel und/oder mit der weiteren flüssigen Phase vorliegt.

18. Verfahren zur Herstellung eines Kits nach einem der Ansprüche 1 bis 17, **dadurch gekennzeichnet, dass** in einem der Verfahrensschritte das Trägermaterial in fester Form in eine Spritze oder in eine Kammer einer Zweikammerspritze gebracht wird.

19. Verfahren zur Herstellung eines Kits nach einem der Ansprüche 1 bis 17, **dadurch gekennzeichnet, dass** in einem der Verfahrensschritte das Trägermaterial als Lösung oder Dispersion in eine Spritze oder in eine Kammer einer Zweikammerspritze gebracht und anschließend darin getrocknet oder lyophilisiert wird.

20. Verfahren zur Herstellung einer Zubereitung mit einer Trägerphase, die im Körper, auf dem Körper oder unter physiologischen Umgebungsbedingungen ein Implantat oder Partikel bildet, wobei die Trägerphase zumindest ein Trägermaterial und ein Lösungsmittel enthält, aus einem Kit nach einem der Ansprüche 1 bis 17, **dadurch gekennzeichnet, dass** das in getrennten Spritzen aufbewahrte Trägermaterial und das Lösungsmittel über ein Verbindungsstück miteinander gemischt werden, so dass sich das Trägermaterial in dem Lösungsmittel löst.

21. Verfahren zur Herstellung einer Zubereitung mit einer Trägerphase, die im Körper, auf dem Körper oder unter physiologischen Umgebungsbedingungen ein Implantat oder Partikel bildet, wobei die Trägerphase zumindest ein Trägermaterial und ein Lösungsmittel enthält, aus einem Kit nach einem der Ansprüche 1 bis 17, **dadurch gekennzeichnet, dass** das in getrennten Kammern einer Zweikammerspritze aufbewahrte Trägermaterial und das Lösungsmittel innerhalb der Zweikammerspritze miteinander gemischt werden, so dass sich das Trägermaterial in dem Lösungsmittel löst.

22. Verfahren zur Herstellung einer Zubereitung nach einem der Ansprüche 20 bis 21, **dadurch gekennzeichnet, dass** die Trägerphase bei oder nach ihrer Herstellung aus dem Trägermaterial und dem Lösungsmittel in einer weiteren flüssigen Phase dispergiert wird.

## Claims

1. A kit for the preparation of a composition comprising a carrier phase, which forms an implant or particles in the body, on the body or under physiological conditions, wherein the carrier phase comprises at least a carrier material and a solvent, wherein the carrier material is a polymer, a lipid or a wax, wherein the carrier material and the solvent are separate from each other in the kit and wherein the carrier material is soluble in the solvent.

2. A kit according to claim 1, wherein the carrier material and the solvent are stored in separate syringes or in separate chambers of a two-chamber syringe, and wherein the carrier material is mixed with the solvent prior to administration through a connector or within the two-chamber syringe and is dissolved therein.

3. A kit according to one of the claims 1 to 2, wherein the carrier material is a water-soluble polymer, a water-insoluble polymer, a polymer, which is soluble in aqueous fluids, a biocompatible and/or biodegradable polymer, a protein, or a combination of these carrier materials.

4. A kit according to claim 3, wherein the carrier material comprises poly(lactide), or a derivative or a copolymer of poly(lactide).

5. A kit according to one of the previous claims, wherein the solvent is water or an organic solvent, such as ethanol, acetone, butanol, ethylformate, acetic acid, pentanol, n-propanol, iso-propanol, tetrahydrofuran, triethylcitrate, triacetin, propylene glycol, glycerol, polyethylene glycol, ethylacetate, methylacetate, ethyllactate, benzylalcohol, dimethylformamide, dimethylsulfoxide, dimethylacetamide, propylene carbonate, glycofurol, oleic acid, 2-pyrrolidone, N-methyl-2-pyrrolidone, an oil, a plasticizer, or a mixture of two or more of these solvents.

6. A kit according to one of the previous claims, wherein the composition comprises at least one active compound.

7. A kit according to claim 6, wherein the active compound is a drug, a peptide- or a protein-drug, an oligonucleotide or a gene therapeutical agent.

8. A kit according to one of the claims 6 to 7, wherein the active compound is a drug belonging to the group of antibiotics (e.g., tetracycline or doxycycline), antiinflammatory agents, antiinfectives, hormones, immunologically active compounds, vaccines, immunomodulators, immunosuppressant, anticancer agents, diuretics, compounds for the gastrointestinal tract, compounds for the heart/circulation, compounds for the respiratory tract, growth factors, analgesics, local anaesthetics and/or neuropharmaceuticals.

9. A kit according to one of the claims 6 to 8, wherein the active compound is present in encapsulated form in the composition.

10. A kit according to one of the claims 6 to 9, wherein the active compound is present in the kit together with the carrier material or is present dissolved and/or dispersed in the solvent or is present with both the carrier material and solvent.

11. A kit according to one of the previous claims, wherein the composition comprises viscosity-modifying substances, stabilizers, release modifying agents, pore-formers, substances, which affect the residence time at the site of administration, bioadhesive materials, penetration enhancers, substances for the retardation of release, and/or substances, which avoid an initial rapid release at the site of administration.

12. A kit according to one of the previous claims, wherein the composition is used for parenteral, peroral, subcutaneous, rectal, buccal, ophthalmic, pulmonal, vaginal, nasal, local, sublingual, periodontal, or transdermal administration, for the placement in body cavities and/or for the application onto body surfaces.

13. A kit according to one of the previous claims, wherein the composition is used for the treatment of soft- and hard tissue defects, for tissue regeneration, as glue, for the filling of body cavities, or for the treatment of wounds.

14. A kit according to one of the previous claims, wherein the composition comprises an additional liquid phase besides the carrier phase, in which the carrier phase is dispersed.

15. A kit according to claim 14, wherein the additional liquid phase is stored separately in the kit from carrier material and the solvent, or in contact with the carrier material and separately from the solvent, or in contact with the solvent and separately from the carrier material, or in a combination of these alternatives.

16. A kit according to one of the claims 14 to 15, wherein the additional liquid phase comprises natural, semisynthetic or synthetic lipids, oils or waxes, such as cottonseed oil, soybean oil, safflower oil, hydrated peanut oil, olive oil, castor oil, triglyceride mixtures (e.g., Miglyol), monoglycerides (e.g., glycerolmonooleate), silicone oil, isopropylmyristate, ethyloleate, paraffins, water, glycerol, propylene glycol, polyethylene glycol or mixtures of two or more of these substances.

17. A kit according to one of the previous claims 14 to 16, wherein at least one active compound is present in the kit together with the carrier material and/or the solvent and/or the additional liquid phase.

18. A method for the preparation of a kit according to one of the claims 1 to 17, wherein the carrier material is placed in solid form in a syringe or in the chamber of a two-chamber syringe in one of the method steps.

19. A method for the preparation of a kit according to one of the claims 1 to 17, wherein the carrier material is placed as solution or as dispersion in a syringe or in the chamber of a two-chamber syringe in one of the method steps and is subsequently dried or lyophilized therein.

20. A method for the preparation of a composition with a carrier phase, which forms an implant or particles in the body, on the body or under physiological conditions, wherein the carrier phase comprises at least one carrier material and one solvent from a kit according to one of the claims 1 to 17, wherein the carrier material and the solvent, which are stored in separate syringes, are mixed through a connector, such that the carrier material dissolves in the solvent.

21. A method for the preparation of a composition with a carrier phase, which forms an implant or particles in the body, on the body or under physiological conditions, wherein the carrier phase comprises at least one carrier material and one solvent from a kit according to one of the claims 1 to 17, wherein the carrier material and the solvent, which are stored in separate chambers of a two-chamber syringe, are mixed within the two-chamber syringe, such that the carrier material dissolves in the solvent.

22. A method for the preparation of a composition according to one of the claims 20 to 21, wherein the carrier phase is dispersed in an additional liquid phase during or after the preparation of the carrier phase from the carrier material and the solvent.

## Revendications

1. Un kit pour la préparation d'une composition comprenant une phase véhicule, qui forme un implant ou des particules dans le corps, sur le corps ou dans des conditions physiologiques, **caractérisé en ce que** la phase véhicule comprend au moins un matériel véhicule et un solvant, **caractérisé en ce que** le matériel véhicule est un polymère, un lipide ou une cire, **caractérisé en ce que** le matériel véhicule et le solvant sont séparés l'un de l'autre dans le kit et **caractérisé en ce que** le matériel véhicule est soluble dans le solvant.

2. Un kit selon la revendication 1, **caractérisé en ce que** le matériel véhicule et le solvant sont conservés dans des seringues séparées ou dans les compartiments séparés d'une seringue à deux compartiments, et **caractérisé en ce que** le matériel véhicule est mélangé avec le solvant à travers un connecteur avant administration ou à l'intérieur d'une seringue à deux compartiments et y est dissout.

3. Un kit selon l'une des revendications 1 à 2, **caractérisé en ce que** le matériel véhicule est un polymère soluble dans l'eau, un polymère insoluble dans l'eau, un polymère soluble dans des fluides aqueux, un polymère biocompatible et/ou biodégradable, une protéine, ou une combinaison de ces matériaux véhicules.

4. Un kit selon la revendication 3, **caractérisée en ce que** le matériel véhicule comprend un polylactide, ou un dérivé ou un copolymère de polylactide.

5. Un kit selon l'une des revendications précédentes, **caractérisé en ce que** le solvant est de l'eau ou un solvant organique tel que l'éthanol, l'acétone, le butanol, l'acide propionique, l'acide acétique, le pentanol, l'alcool propylique normal, l'alcool isopropylique, le tétrahydrofurane, le citrate de triéthyle, la triacétine, le propylèneglycol, le glycérol, les polyéthylèneglycols, l'acétate d'éthyle, l'acétate de méthyle, le lactate éthylique, l'alcool benzylique, le diméthylformamide, le diméthylsulfoxyde, le diméthylacétamide, le carbonate de propylène, le glycofurol, l'acide oléique, le 2-pyrrolidone, le N-Méthyl-2-pyrrolidone, une huile, un plastifiant, ou un mélange de deux ou plus de ces solvants.

6. Un kit selon l'une des revendications précédentes, **caractérisé en ce que** la composition comprend au moins une substance active.

7. Un kit selon la revendication 6, **caractérisé en ce que** la substance active est un principe actif, un peptide-principe actif ou une protéine-principe actif, un oligonucléotide ou un agent thérapeuthique génique.

8. Un kit selon l'une des revendications 6 à 7, **caractérisé en ce que** la substance active est un principe actif appartenant au groupe des antibiotiques (par exemple tétracycline ou doxycycline), des agents anti-inflammatoires, des anti-infectieux, des hormones, des composés immunologiquement actifs, des vaccins, des immunomodulateurs, des immunosuppresseurs, des agents anticancéreux, des diurétiques, des substances pour l'appareil gastro-intestinal, des substances pour l'appareil cardiovasculaire, des substances pour l'appareil respiratoire, des facteurs de croissance, des analgésiques, des anesthésiques locaux et/ou des agents neuropharmaceutiques.

9. Un kit selon l'une des revendications 6 à 8, **caractérisé en ce que** la substance active est présente sous forme encapsulée dans la composition.

10. Un kit selon l'une des revendications 6 à 9, **caractérisé en ce que** la substance active est présente dans le kit mélangée avec le matériel véhicule ou est présente dissoute et/ou dispersée dans le solvant ou est présente avec le matériel véhicule et le solvant ensemble.

11. Un kit selon l'une des revendications précédentes, **caractérisé en ce que** la composition comprend des substances modifiant la viscosité, des agents stabilisants, des agents modifiant la libération, des substances créant des pores, des substances qui modifient le temps de résidence au niveau du site d'administration, des matériaux bioadhésifs, des promoteurs d'absorption, des substances retardant la libération, et/ou des substances qui évitent une libération initiale rapide au niveau du site d'administration.

12. Un kit selon l'une des revendications précédentes, **caractérisé en ce que** la composition est utilisée pour l'administration parentérale, *per os,* sous-cutanée, rectale, buccale, ophtalmique, pulmonaire, vaginale, nasale, locale, sublinguale, périodontale ou transdermique, pour le placement dans des cavités du corps et/ou pour l'application sur les surfaces du corps.

13. Un kit selon l'une des revendications précédentes, **caractérisé en ce que** la composition est utilisée pour le traitement de défauts des tissus mous et des tissus durs, pour la régénération tissulaire, comme colle, pour le remplissage de cavités du corps, ou pour le traitement de blessures.

14. Un kit selon l'une des revendications précédentes, **caractérisé en ce que** la composition comprend une phase liquide additionnelle en plus de la phase véhicule, dans laquelle la phase véhicule est dispersée.

15. Un kit selon la revendication 14, **caractérisé en ce que** la phase liquide additionnelle est conservée dans le kit séparément du matériel véhicule et du solvant, ou en contact avec le matériel véhicule et séparément du solvant, ou en contact avec le solvant et séparément du matériel véhicule, ou dans une combinaison de ces alternatives.

16. Un kit selon l'une des revendications 14 à 15, **caractérisé en ce que** la phase liquide additionnelle comprend des lipides naturels, semi-synthétiques ou synthétiques, des huiles ou des cires, comme l'huile de coton, l'huile de soja, l'huile de carthame, l'huile d'arachide hydrogénée, l'huile d'olive, l'huile de ricin, les mélanges de triglycérides (par exemple Miglyol), les monoglycérides (par exemple monooléate de glycérol), les siloxanes, le myristate d'isopropyle, l'oléate d'éthyle, les paraffines, l'eau, le glycérol, le propylèneglycol, les polyéthylèneglycols ou des mélanges de deux ou plus de ces substances.

17. Un kit selon l'une des revendications précédentes 14 à 16, **caractérisé en ce qu'**au moins une substance active est présente dans le kit avec le matériel véhicule et/ou le solvant et/ou la phase liquide additionnelle.

18. Une méthode pour la préparation d'un kit selon l'une des revendications 1 à 17, **caractérisé en ce que** le matériel véhicule est placé sous forme solide dans une seringue ou dans un compartiment d'une seringue à deux compartiments dans une des étapes de la méthode.

19. Une méthode pour la préparation d'un kit selon l'une des revendications 1 à 17, **caractérisé en ce que** le matériel véhicule est placé sous forme de solution ou de dispersion dans une seringue ou dans un compartiment d'une seringue à deux compartiments dans une des étapes de la méthode et y est ensuite séché ou lyophilisé.

20. Une méthode pour la préparation d'une composition avec une phase véhicule qui forme un implant ou des particules dans le corps, sur le corps ou dans des conditions physiologiques, **caractérisée en ce que** la phase véhicule comprend au moins un matériel véhicule et un solvant d'un kit selon l'une des revendications 1 à 17, **caractérisée en ce que** le matériel véhicule et le solvant, qui sont conservés dans des seringues séparées, sont mélangés à travers un connecteur, de telle sorte que le matériel véhicule se dissolve dans le solvant.

21. Une méthode pour la préparation d'une composition avec une phase véhicule qui forme un implant ou des particules dans le corps, sur le corps ou dans des conditions physiologiques, **caractérisée en ce que** la phase véhicule comprend au moins un matériel véhicule et un solvant d'un kit selon l'une des revendications 1 à 17, **caractérisée en ce que** le matériel véhicule et le solvant, qui sont conservées dans les compartiments séparées d'une seringue à deux compartiments, sont mélangés dans la seringue à deux compartiments, de telle sorte que le matériel véhicule se dissolve dans le solvant.

22. Une méthode pour la préparation d'une composition selon l'une des revendications 20 à 21, **caractérisée en ce que** la phase véhicule est dispersée dans une phase liquide additionnelle pendant ou après la préparation de la phase véhicule à partir du matériel véhicule et du solvant.
